(19) **Europäisches Patentamt / European Patent Office / Office européen des brevets**

(11) **EP 1 479 084 B1**

(12) **EUROPÄISCHE PATENTSCHRIFT**

(45) Veröffentlichungstag und Bekanntmachung des Hinweises auf die Patenterteilung:
**20.08.2008 Patentblatt 2008/34**

(51) Int Cl.:
**G21G 4/08** (2006.01)

(21) Anmeldenummer: **03706286.6**

(22) Anmeldetag: **06.02.2003**

(86) Internationale Anmeldenummer:
**PCT/DE2003/000332**

(87) Internationale Veröffentlichungsnummer:
**WO 2003/073437 (04.09.2003 Gazette 2003/36)**

(54) **VORRICHTUNG SOWIE VERFAHREN ZUR ABTRENNUNG VON TRÄGERFREIEN RADIONUKLIDEN UND DEREN RADIOCHEMISCHE UMSETZUNG**

APPARATUS, METHOD FOR SEPARATING CARRIER-FREE RADIONUCLIDES, AND THE RADIOCHEMICAL REACTION THEREOF

DISPOSITIF ET PROCEDE DE SEPARATION DE RADIONUCLEIDES DEPOURVUS DE PORTEURS, ET LEUR CONVERSION RADIOCHIMIQUE

(84) Benannte Vertragsstaaten:
**AT BE BG CH CY CZ DE DK EE ES FI FR GB GR HU IE IT LI LU MC NL PT SE SI SK TR**

(30) Priorität: **28.02.2002 DE 10208668**

(43) Veröffentlichungstag der Anmeldung:
**24.11.2004 Patentblatt 2004/48**

(73) Patentinhaber: **Forschungszentrum Jülich GmbH 52425 Jülich (DE)**

(72) Erfinder:
• **HAMACHER, Kurt**
  **52076 Aachen (DE)**
• **BOLTEN, Willi**
  **52428 Jülich (DE)**

(56) Entgegenhaltungen:
**DE-A- 10 108 440**      **US-A- 5 770 030**

• **DATABASE WPI Section Ch, Week 200236 Derwent Publications Ltd., London, GB; Class A35, AN 2002-318832 XP002242802 & JP 2001 281131 A (TOA IYO DENSHI KK), 10. Oktober 2001 (2001-10-10)**

**EP 1 479 084 B1**

**Beschreibung**

Die Erfindung betrifft eine Vorrichtung sowie ein Verfahren zur Abtrennung von trägerfreien Radionukliden aus flüssigem bzw. verflüssigbarem Targetmaterial und deren radiochemische Umsetzung.

Radionuklide können durch Kernumwandlungsprozesse in einem Zyklotron z. B. durch Bestrahlung eines geeigneten Targets mit Protonen hergestellt werden. Eine genaue Beschreibung eines Verfahrens zur Abtrennung von trägerfreien Radionukliden aus einer Targetflüssigkeit ist beispielsweise aus DE 195 00 428 bekannt.

Aus DE 195 00 428 ist ebenfalls eine Vorrichtung (Durchflusszelle) zur Abtrennung trägerfreier Radionuklide aus einer Targetflüssigkeit bekannt, die im wesentlichen aus einem Zylinder aus Glaskohlenstoff (Sigradur®) und einer axialen Platinkanüle, durch die hindurch ein zylinderförmiges Gefäß gefüllt oder entleert wird bzw. durch die Inertgas in die Kammer eingeleitet werden kann, besteht. Das System wird durch eine Kunststoffhalterung fixiert und abgedichtet. Am unteren Ende ist ein flacher Trichter eingearbeitet, der in die das Wasser abführende Leitung einmündet. Im Kopfteil der Halterung befindet sich eine Gaszuleitung sowie eine Öffnung, durch die Gas abgeleitet werden kann. Der Zylinder aus Glaskohlenstoff (Sigradur®) und die Platinkanüle sind an eine Gleichstromquelle angeschlossen und können wahlweise als Kathode oder Anode geschaltet werden. Zur Gewinnung der gewünschten Radionuklide, z. B. [$^{18}$F]Fluorid aus [$^{18}$O]H$_2$O wird die Durchflusszelle mit dem [$^{18}$F]Fluorid-haltigen Targetwasser befüllt. Das [$^{18}$F]Fluorid wird anodisch an der Oberfläche des Zylinders abgeschieden und das $^{18}$O-Wasser heraustransportiert. Die Höhe der Zone des anodisch fixierten [$^{18}$F]Fluorids ist identisch mit dem Füllstand des $^{18}$O-Wassers in der Zelle. Damit das an der Oberfläche fixierte Radioisotop durch Umpolung des elektrischen Feldes vollständig in eine andere flüssige Phase überführt werden kann, ist es notwendig, den Füllstand in der Zelle dem des $^{18}$O-Wassers anzupassen. Für die nachfolgende $^{18}$F-Fluorierung muß daher ein Reaktionsvolumen zugegeben werden, das dem des Targetwassers entspricht. Daraus entsteht die Notwendigkeit, die Stoffmenge der Edukte, entsprechend der optimalen Eduktkonzentration, diesem Volumen (z. B. 1,3 ml) anzupassen. Aufgrund des sehr geringen Stoffmengenanteils des Produkts (picobis nanomol) gegenüber dem Anteil des nicht umgesetzten Edukts (μmol) führt dies bei der Aufreinigung, insbesondere bei der chromatographischen Aufreinigung, zu erschwerten Trennungsbedingungen. Da die Masse des Eduktes die Masse des Produktes wesentlich überschreitet, verläuft eine HPL-chromatographische Trennung i. a. nur mit schlechter Auflösung.

Das US-Patent 5,770,030 offenbart eine Vorrichtung zur Abtrennung von trägerfreien Radionukliden aus flüssigem bzw. verflüssigbarem Targetmaterial und deren radiochemische Umsetzung. Aus der japanischen Schrift 2001-281131 ist eine Durchflusszelle bekannt, die einen bewegbaren Kolben aufweist, der gegenüber eines sich darin beweglichen Zylinders einen Spalt aufweist.

Es ist daher Aufgabe der Erfindung eine Vorrichtung und ein Verfahren zu schaffen, mit der das Reaktionsvolumen vermindert werden kann. Es ist weiterhin Aufgabe der Erfindung die Stoffmenge des Edukts unter Beibehaltung der optimalen Eduktkonzentration um ein Mehrfaches zu verringern.

Ausgehend vom Oberbegriff des Anspruchs 1 wird die Aufgabe erfindungsgemäß gelöst mit den im kennzeichnenden Teil des Anspruchs 1 angegebenen Merkmalen. Die Aufgabe wird weiterhin gelöst durch die im kennzeichnenden Teil des Anspruchs 14 angegebenen Merkmale.

Mit der erfindungsgemäßen Vorrichtung und dem Verfahren ist es nunmehr möglich, durch die Reduktion des Zylindervolumens (= Reaktionsvolumen), das Reaktionsvolumen und damit auch die Eduktmenge um ein Mehrfaches zu verringern. Die Verminderung der Eduktmenge vereinfacht die chromatographische Reinigung und ermöglicht eine quantitative Abtrennung der trägerarm markierten Verbindungen (Produkt) vom Edukt. Da geringere Eduktmengen eingesetzt werden können, werden die Kosten reduziert und die Wirtschaftlichkeit des Verfahrens erhöht.

Vorteilhafte Weiterbildungen sind in den Unteransprüchen angegeben.

Die Zeichnungen zeigen eine beispielhafte Ausführungsform des erfindungsgemäßen Verfahrens und der Vorrichtung.

Es zeigt:

Fig. 1: Querschnitt Vorrichtung in Position I

Fig. 2: Querschnitt Vorricthung in Position II

Fig. 3. Aufsicht Vorricthtung in Position II durch Schnittebene A

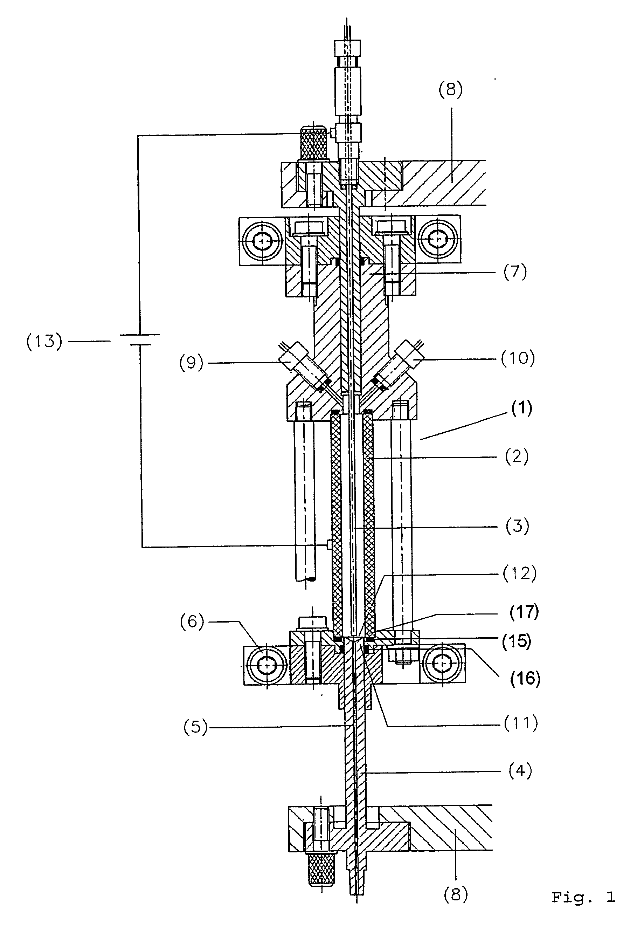

Figur 1 zeigt die Vorrichtung 1 mit einem Zylinder 2 mit eingelassener Kanüle 3, die von unten durch einen Kolben 4 mit Bohrung 5, der über ein Joch 6 mit einer Kanülenhalterung 7 verbunden ist, begrenzt wird. Der Zylinder 2 kann durch die Kanüle 3 mit Flüssigkeit oder mit Inertgas gefüllt werden. Die Vorrichtung 1 wird durch eine Halterung 8 fixiert und abgedichtet. Im Kopfteil der Halterung 8 befinden sich zwei Zuleitungen 9 und 10 über die Gase zu oder abgeführt werden können. In den Kopf des Kolbens 11 ist ein flacher Trichter 12 eingearbeitet, der in eine Bohrung 5 einmündet. Der Zylinder 2 und die Kanüle 3 sind an eine Gleichstromquelle 13 angeschlossen und können wahlweise

2

als Kathode oder Anode geschaltet werden. In Position I befindet sich die Kanüle 3 am unteren Ende des Zylinders 2. Der Kolben 4 befindet sich außerhalb des Zylinders 2. Im unteren Bereich 17 des Zylinders dichten zwei Dichtungsringe 15 und 16 den Kolben 4 gegen den Zylinder 2 ab.

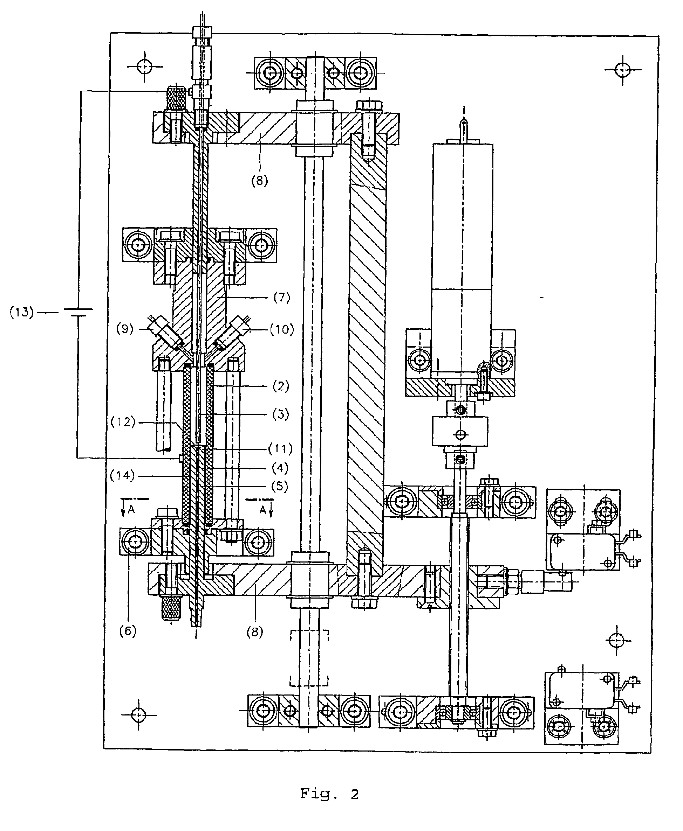

In Fig. 2 sind denselben Vorrichtungsmerkmalen dieselben Bezugzeichen zugeordnet. Fig. 2 zeigt die Anordnung der Komponenten der Vorrichtung 1 in Position II. In Position II ist der Kolben 4 in den Zylinder 2 hinein geschoben. Die Kanüle 3 ist entsprechend der Höhe, mit der der Kolben 4 in den Zylinder 2 hineinragt, aus dem Zylinder 2 herausgeschoben. Zwischen Kolben 4 und Zylinder 2 hat sich ein ringförmiger Spalt 14 gebildet. In Figur 2 ist im unteren Bereich des Zylinders durch die Kennzeichnung "A" eine Schnittebene markiert.

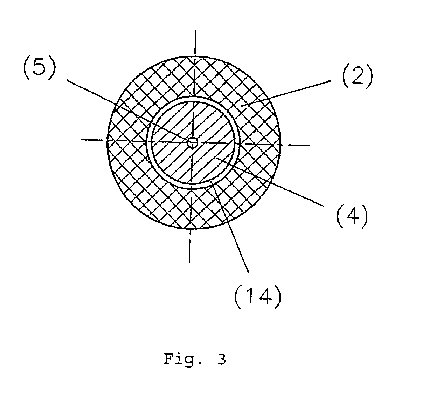

Figur 3 zeigt eine Aufsicht auf die Vorrichtung 1 in Position II durch die Schnittebene A. Zwischen Kolben 4 und Zylinder 2 ist der ringförmige Spalt 14 zu sehen.

Im Folgenden soll die Erfindung beispielhaft beschrieben werden.

Zu Beginn der Reaktion können die Komponenten der Vorrichtung 1 beispielsweise wie folgt (s. auch Figur 1; Position I) angeordnet sein: die untere Öffnung der Kanüle 3, welche vorzugsweise aus Platin gefertigt ist, befindet sich am unteren Ende 17 des Zylinders 2 und der Kolben 4 befindet sich außerhalb des Zylinders 2. Die Vorrichtung 1 wird über die Bohrung 5 oder die Leitung 3 mit dem [18F]Fluorid-haltigen Targetwasser gefüllt, wobei anschließend das [18F]Fluorid anodisch an der Oberfläche des Zylinders 2 abgeschieden wird, indem an diesen über eine Gleichspannungsquelle 13 eine Gleichspannung von beispielsweise 20 V angelegt wird. Die Kanüle 3 dient hier als Kathode. Der Zylinder 2 ist in einer vorteilhaften Ausgestaltung der Vorrichtung aus porenfreiem, inertem Material wie z. B. Glaskohlenstoff (Sigradur®), Edelmetall oder Platin gefertigt.

Der Kolben 4 sollte bevorzugt aus inertem Material gefertigt werden. Als geeignet hat sich beispielsweise PEEK (Polyetheretherketon), Quarzglas oder Geräteglas erwiesen. Es ist jedoch auch möglich einen Kolben aus elektrisch leitfähigem Material zu verwenden, der dann auch als Elektrode eingesetzt werden kann. Die Spaltbreite bzw. Radiendifferenz zwischen Zylinder 2 und Kolben 4 ist von den Fertigungsmethoden abhängig. Als bevorzugt hat sich eine Radiendifferenz zwischen Zylinder 2 und Kolben 4 von 0,4 mm erwiesen. Geeignet sind aber auch Spaltbreiten von $\leq 0,2$ mm. Für das Verhältnis der Radien zwischen Zylinder ($r_1$) und Kolben ($r_2$) können folgende Gleichungen angegeben werden:

$$F_1 \;=\; r_1{}^2 \;*\; \pi$$

mit $F_1$ = Fläche des Zylinders 2

$$F_2 \;=\; r_2{}^2 \;*\; \pi$$

mit $F_2$ = Fläche des Kolbens 4

$$F_3 \;=\; F_1 \;-F_2 \;=\; \pi(r_1{}^2 - r_2{}^2)$$

mit $F_3$ = Fläche des Spalts 14

$$V_3 \;\approx\; r_1{}^2 - r_2{}^2 \;\approx\; (r_1 + r_2)\,(r_1 - r_2)$$

mit $V_3$ = Volumen des Spalts 14

$$\frac{V_1}{V_3} = \frac{r_1^2}{r_1^2 - r_2^2} = \frac{r_1^2}{(r_1 + r_2)(r_1 - r_2)}$$

mit $V_1$ = Volumen des Zylinders 2

Nach dem Befüllen der Vorrichtung 1 mit der Targetlösung (z. B $^{18}$O-Wasser) wird das Radioisotop (z. B. [$^{18}$F] Fluorid) auf der inneren Oberfläche des Zylinders 2 abgeschieden und das $^{18}$O-Wasser durch die Bohrung 5 im Kolben 4 aus der Vorrichting 1 heraus transportiert. Die Höhe der Zone des anodisch fixierten [$^{18}$F]Fluorids entspricht dem Füllstand des $^{18}$O-Wassers in dem Zylinder 2. Das an der Oberfläche des Zylinders 2 fixierte [$^{18}$F]Fluorid wird anschließend durch Umpolung des elektrischen Feldes vollständig in eine andere flüssige Phase wie z. B. eine organische Phasentransferkatalysator-haltige Lösung ([K$\subset$2.2.2]$_2$C$_2$O$_4$ in Dimethylsulfoxid= DMSO) überführt. Dazu ist es notwendig, den Füllstand dieser flüssigen Phase in dem Zylinder 2 dem vorher eingestellten Füllstand des $^{18}$O-Wassers anzupassen. Um zu vermeiden, dass für die nachfolgende nukleophile [$^{18}$F]-Fluorierung ein dem Targetwasser entsprechendes Reaktionsvolumen zugegeben werden muss, wird der Verdrängungseffekt des Kolbens 4 genutzt. Der Kolben 4 wird durch die Verschiebung des Jochs 6 nach oben transportiert. Gleichzeitig wird die Kanüle 3 entsprechend der Höhe mit der der Kolben 4 in den Zylinder 2 geschoben wird, aus den Zylinder 2 herausgeschoben. Das Volumen, das nun in den Zylinder 2 eingefüllt werden muss, entspricht dem Volumen des Spalts 14, das sich ergibt, wenn der Kolben 4 soweit in die Vorrichtung 1 ragt (s. Fig. 2, Position II), dass das obere Ende des Kolbens 4 mit dem Füllstand des an der Oberfläche des Zylinders 2 fixierten [$^{18}$F]Fluorids übereinstimmt. In einer vorteilhaften Ausgestaltung der Vorrichtung beträgt das Verhältnis des Volumens ($V_1$) des Zylinders 2 zum Volumen ($V_3$) des Spaltes 14 etwa 4:1 bis 10:1. In einer besonders bevorzugten Ausgestaltung der Vorrichtung beträgt es 4:1. Bei einem $^{18}$O-Wasservolumen von beispielsweise 1,3 ml beträgt dann das Volumen des Spaltes 14 bei identischem Füllstand etwa 0,29 ml. Wenn der Kolben 4 so weit nach oben transportiert wurde, dass das obere Ende des Kolbens 4 mit dem Füllstand des an der Oberfläche des Zylinders fixierten [$^{18}$F]Fluorids übereinstimmt (s. Fig.2, Position B), kann das [$^{18}$F]Fluorid durch Anlegen eines elektrischen Feldes (Sigradur® als Kathode) in die Reaktionslösung überführt werden. In dieser Position muß nur das Spaltvolumen 14 ausgefüllt werden. Der Zylinder wird im unteren Ende 17 durch zwei ringförmige Dichtungen 15 und 16 (z. B. O-Ringe) gegen den Kolben 4 abgedichtet, so dass ein seitliches Auslaufen von Flüssigkeiten am unteren Ende 17 des Zylinders verhindert wird.

Zum Entleeren des Reaktionsgefäßes wird der Kolben 4 wieder nach unten abgesenkt und die Flüssigkeit entweder durch die Kanüle 3 oder die Bohrung 5 des Kolbens 4 nach außen transportiert.

In einer vorteilhaften Ausgestaltung der Vorrichtung und des Verfahrens erfolgt die Zufuhr bzw. das Absaugen des [$^{18}$F]Fluorid-haltigen Targetwassers und des organischen Lösungsmittels durch unterschiedliche Leitungen. Wenn beispielsweise die Zufuhr des [$^{18}$F]Fluorid-haltigen Targetwassers durch die Leitung 3 erfolgt, sollte der Transport des organischen Lösungsmittels, welches zur Desorption des Produktes eingesetzt wird, durch die Bohrung 5 erfolgen, um eine Verunreinigung der Leitung 3 zu vermeiden. So kann eine arbeitsaufwendige Reinigung der Leitung für das Targetwasser umgangen werden. Der Transport des Targetwassers kann selbstverständlich auch durch die Bohrung 5 erfolgen und der Transport des organischen Lösungsmittels entsprechend durch die Leitung 3. Wichtig für eine einfach zu handhabende Vorrichtung und das Verfahren ist eine getrennte Leitung für die unterschiedlichen Flüssigkeiten.

Durch das erfindungsgemäße Verfahren und die Vorrichtung ist es möglich, die Eduktmenge zu reduzieren. Diese Verminderung der Masse ist besonders für die folgende chromatographische Trennung des trägerarmen $^{18}$F-markierten Produktes vom Edukt vorteilhaft. Bei bisher bekannten Verfahren und Vorrichtungen zur Abtrennung der trägerfreien Radionuklide und deren radiochemische Umsetzung ist die Eduktmenge deutlich größer. Durch das erfindungsgemäße Verfahren und die Vorrichtung wird das Volumen der Eduktlösung und damit die Menge des Eduktes um ein Mehrfaches (mindestens 3 bis 4-faches) reduziert, wodurch die chromatographische Separation von Edukt und dem $^{18}$F-markierten Produkt wesentlich verbessert wird.

Die Verminderung der absoluten Eduktmenge führt darüber hinaus zu einer Kostenersparnis und damit zur Verbesserung der Wirtschaftlichkeit des Verfahrens.

Ausführungsbeispiel:

### $^{18}$F-Markierung von N-Methylbenperidol

Das vom Target des Zyklotrons kommende [$^{18}$F]Fluoridhaltige $^{18}$O-Wasser (1,3 ml) wird durch die Bohrung 5 des Kolbens 4 in die Vorrichtung 1 transportiert. Durch Anlegen einer elektrischen Spannung von 20 V über die Gleichstromquelle 13 (Anode = Zylinder (2) aus Glaskohlenstoff) wird das trägerarme Radioisotop innerhalb von etwa 8 min. an der Oberfläche des Zylinders 2 anodisch adsorbiert. Das $^{18}$O-Wasser wird durch die Bohrung 5 mittels Helium nach außen gedrückt. Zur Trocknung der Zylinderwandung wird die Vorrichtung 1 - 2-mal mit je 1,6 ml wasserfreiem Dimethylsulfoxid (DMSO) gefüllt und durch die als Gegenelektrode dienende Kanüle 3 entleert. Durch die Kanüle 3 wird eine Lösung von 5 mg Kryptat ([K⊂2.2.2]$_2$CO$_3$ in 300 µl DMSO) in die Vorrichtung 1 eingefüllt und der Kolben 4 motorisch soweit verschoben, dass die Flüssigkeitsoberfläche die obere Grenze des anodisch abgeschiedenen [$^{18}$F]Fluorids erreicht. Zur Desorption des [$^{18}$F]Fluorids wird die Spannung umgepolt (-2V) und der Zylinder 5 min auf etwa 100°C erwärmt. Nach Depolarisation der Vorrichtung wird der Kolben in die untere Position gebracht und durch die Kanüle 3 eine Lösung von 2 mg N-Methyl-desfluor-nitro-benperidol in 150 µl DMSO zugegeben. Der Kolben 4 wird erneut in die obere Position geschoben und die Vorrichtung 10 min. auf 150°C erwärmt. Anschließend wird mit Druckluft auf Umgebungs temperatur abgekühlt. Die Vorrichtung wird durch die Kanüle 3 entleert, mit etwa 0,8 ml Acetonitril bei 50°C gewaschen und diese Lösung mit der DMSO-Produktlösung vermischt. Durch nachfolgende HPL-Chomatographie wird der Radiotracer [$^{18}$F]N-Methylbenperidol gereinigt und isoliert.

**Patentansprüche**

1. Vorrichtung (1) Speziellausgebilldet zur Abtrennung von trägerfreien Radionukliden aus flüssigem bzw. verflüssigbarem Targetmaterial und für deren radiochemische Umsetzung, umfassend einen Zylinder (2), **gekennzeichnet durch** einen Kolben (4) und **durch** einen ringförmigen Spalt (14) zwischen Zylinder (2) und Kolben (4).

2. Vorrichtung (1) nach Anspruch 1,
   **dadurch gekennzeichnet**, der Kolben (4) einen Kreisförmigen Querschnitt hat, und
   **dass** der Durchmesser des Kolbens (4) kleiner als der Durchmesser des Zylinders (2) ist.

3. Vorrichtung (1) nach einem der Ansprüche 1 bis 2,
   **dadurch gekennzeichnet,**
   **dass** das Volumenverhältnis zwischen Zylinder (2) und Spalt (14) 4:1 bis 10:1 beträgt.

4. Vorrichtung (1) nach einem der Ansprüche 1 bis 3,
   **dadurch gekennzeichnet,**
   **dass** der Kolben (4) chemisch inertes Material umfasst.

5. Vorrichtung (1) nach einem der Ansprüche 1 bis 4,
   **dadurch gekennzeichnet,**
   **dass** der Kolben (4) PEEK, Geräteglas, Quarzglas oder elektrisch leitfähiges Material umfasst.

6. Vorrichtung (1) nach einem der Ansprüche 1 bis 5,
   **dadurch gekennzeichnet,**
   **dass** der Kolben (4) eine Bohrung (5) aufweist.

7. Vorrichtung (1) nach einem der Ansprüche 1 bis 6,
   **dadurch gekennzeichnet,**
   **dass** der Kolben (4) eine zentrierte Bohrung (5) aufweist.

8. Vorrichtung nach einem der Ansprüche 1 bis 7,
   **dadurch gekennzeichnet,**
   **dass** der Zylinder (2) eine porenfreie inerte Oberfläche aufweist.

9. Vorrichtung (1) nach einem der Ansprüche 1 bis 8,
   **dadurch gekennzeichnet,**
   **dass** der Zylinder (2) chemisch inertes Material umfasst.

10. Vorrichtung (1) nach einem der Ansprüche 1 bis 9,
    **dadurch gekennzeichnet,**
    **dass** der Zylinder (2) Glaskohlenstoff, Edelmetall oder Platin umfasst.

11. Vorrichtung (1) nach einem der Ansprüche 1 bis 10,
    **dadurch gekennzeichnet,**
    **dass** die Fläche des Zylinders (2) im Betrieb elektrisch geladen ist.

**12.** Vorrichtung (1) nach einem der Ansprüche 1 bis 11,
**dadurch gekennzeichnet,**
**dass** sie eine Kanüle (3) umfasst, welche in dem Zylinder verschiebbar ist und elektrisch geladen ist.

**13.** Vorrichtung (1) nach Anspruch 12, wenn abhängend von Anspruch 11,
**dadurch gekennzeichnet,**
**dass** die Kanüle (3) die Gegenelektrode zum elektrisch geladenen Zylinder (2) ist.

**14.** Verfahren zur Abtrennung von trägerfreien Radionukliden aus flüssigem bzw. verflüssigbarem Targetmaterial und deren radiochemische Umsetzung,
**dadurch gekennzeichnet,**
**dass** eine Vorrichtung (1) nach einem der Ansprüche 1 bis 13 eingesetzt wird.

**15.** Verfahren nach Anspruch 14,
**dadurch gekennzeichnet,**
**dass** nach Fixierung des Radionuklids auf der Oberfläche des Zylinders (2) der Kolben (4) in den Zylinder geschoben wird und gleichzeitig die Kanüle (3) entsprechend aus dem Zylinder heraus geschoben wird.

**16.** Verfahren nach einem der Ansprüche 14 bis 15,
**dadurch gekennzeichnet,**
**dass** das obere Ende des Kolbens (4) bis zum Füllstand des Targetmaterials im Zylinder (2) geschoben wird.

**17.** Verfahren nach einem der Ansprüche 14 bis 16,
**dadurch gekennzeichnet**
**dass** das Volumen des ringförmigen Spaltes (14) zwischen Kolben (4) und Zylinder (2) mit einer flüssigen Phase gefüllt wird.

**18.** Verfahren nach Anspruch 17,
**dadurch gekennzeichnet,**
**dass** nach Desorption des Radionuklids in die flüssige Phase der Kolben (4) aus dem Zylinder (2) heraus geschoben wird.

**Claims**

**1.** Apparatus (1) specially designed for separating carrier-free radionuclides from liquid or liquefiable target material and for the radiochemical reaction thereof, comprising a cylinder (2) **characterised by** a piston (4) and by an annular gap (14) between the cylinder (2) and the piston (4).

**2.** Apparatus (1) according to claim 1,
**characterised in that** the piston (4) has a circular cross-section and that the diameter of the piston (4) is smaller than the diameter of the cylinder (2).

**3.** Apparatus (1) according to one of claims 1 to 2,
**characterised in that**
the volume ratio between the cylinder (2) and the gap (14) amounts to 4:1 to 10:1.

**4.** Apparatus (1) according to one of claims 1 to 3,
**characterised in that**
the piston (4) comprises a chemically inert material.

**5.** Apparatus (1) according to one of claims 1 to 4,
**characterised in that**
the piston (4) comprises PEEK, laboratory glass, vitreous silica or electrically conductive material.

**6.** Apparatus (1) according to one of claims 1 to 5,
**characterised in that**
the piston (4) has a bore (5).

**7.** Apparatus (1) according to one of claims 1 to 6,
**characterised in that**
the piston (4) has a centred bore (5).

**8.** Apparatus (1) according to one of claims 1 to 7,
**characterised in that**
the cylinder (2) has a pore-free inert surface.

**9.** Apparatus (1) according to one of claims 1 to 8,
**characterised in that**
the cylinder (2) comprises a chemically inert material.

**10.** Apparatus (1) according to one of claims 1 to 9,
**characterised in that**
the cylinder (2) comprises glassy carbon, noble metal or platinum.

**11.** Apparatus (1) according to one of claims 1 to 10,
**characterised in that**
the surface of the cylinder (2) is electrically charged during operation.

**12.** Apparatus (1) according to one of claims 1 to 11,
**characterised in that**
it comprises a cannula (3), which may be slid into the cylinder and is electrically charged.

**13.** Apparatus (1) according to claim 12 when dependent on claim 11,
**characterised in that**
the cannula (3) is the counter electrode to the electrically charged cylinder (2).

**14.** Method for separating carrier-free radionuclides from liquid or liquefiable target material and for the radiochemical reaction thereof,
**characterised in that**
apparatus (1) is used according to one of claims 1 to 13.

**15.** Method according to claim 14
**characterised in that**
following the fixing of the radionuclide on the surface of the cylinder (2) the piston (4) is slid into the cylinder and at the same time the cannula (3) is correspondingly slid out of the cylinder.

**16.** Method according to one of claims 14 to 15
**characterised in that**
the top end of the piston (4) is slid up to the fill level of the target material in the cylinder (2).

**17.** Method according to one of claims 14 to 16
**characterised in that**
the volume of the annular gap (14) between the piston (4) and the cylinder (2) is filled with a liquid phase.

**18.** Method according to claim 17,
**characterised in that**
following desorption of the radionuclide in the liquid phase the piston (4) is slid out from the cylinder (2).

**Revendications**

**1.** Dispositif (1) spécialement conçu pour la séparation de radionucléides dépourvus de porteurs à partir d'une matière cible liquide ou liquéfiable et pour leur conversion radiochimique, comportant un cylindre (2), **caractérisé par** un piston (4) et par une fente (14) annulaire entre le cylindre (2) et le piston (4).

**2.** Dispositif (1) selon la revendication 1, **caractérisé en ce que** le piston (4) a une section circulaire et **en ce que** le diamètre du piston (4) est plus petit que le diamètre du cylindre (2).

3.  Dispositif (1) selon l'une quelconque des revendications 1 à 2, **caractérisé en ce que** le rapport de volume entre le cylindre (2) et la fente (14) est de l'ordre de 4:1 à 10:1.

4.  Dispositif (1) selon l'une quelconque des revendications 1 à 3, **caractérisé en ce que** le piston (4) est réalisé dans un matériau chimiquement inerte.

5.  Dispositif (1) selon l'une quelconque des revendications 1 à 4, **caractérisé en ce que** le piston (4) est réalisé en PEEK, en verre pour la fabrication d'appareils, en verre de quartz ou dans un matériau électroconducteur.

6.  Dispositif (1) selon l'une quelconque des revendications 1 à 5, **caractérisé en ce que** le piston (4) comporte une forure (5).

7.  Dispositif (1) selon l'une quelconque des revendications 1 à 6, **caractérisé en ce que** le piston (4) comporte une forure (5) centrée.

8.  Dispositif (1) selon l'une quelconque des revendications 1 à 7, **caractérisé en ce que** le cylindre (2) comporte une surface inerte sans pores.

9.  Dispositif (1) selon l'une quelconque des revendications 1 à 8, **caractérisé en ce que** le cylindre (2) est réalisé dans un matériau chimiquement inerte.

10. Dispositif (1) selon l'une quelconque des revendications 1 à 9, **caractérisé en ce que** le cylindre (2) est réalisé en carbone vitreux, dans un métal inoxydable ou en platine.

11. Dispositif (1) selon l'une quelconque des revendications 1 à 10, **caractérisé en ce que** la surface du cylindre (2) est chargée électriquement en cours de service.

12. Dispositif (1) selon l'une quelconque des revendications 1 à 11, **caractérisé en ce qu'**il comporte une canule (3), qui peut coulisser dans le cylindre et est chargée électriquement.

13. Dispositif (1) selon la revendication 12, lorsqu'il dépend de la revendication 11, **caractérisé en ce que** la canule (3) est la contre-électrode du cylindre (2) chargé électriquement.

14. Procédé de séparation de radionucléides dépourvus de porteurs à partir d'une matière cible liquide ou liquéfiable et leur conversion radiochimique, **caractérisé en ce qu'**il utilise un dispositif (1) selon l'une quelconque des revendications 1 à 13.

15. Procédé selon la revendication 14, **caractérisé en ce que,** après la fixation des radionucléides sur la surface du cylindre (2), le piston (4) est déplacé dans le cylindre et, en même temps, la canule (3) est déplacée de manière correspondante hors du cylindre.

16. Procédé selon l'une quelconque des revendications 14 à 15, **caractérisé en ce que** l'extrémité supérieure du piston (4) est déplacée dans le cylindre (2) jusqu'au niveau de remplissage de la matière cible.

17. Procédé selon l'une quelconque des revendications 14 à 16, **caractérisé en ce que** le volume de la fente (14) annulaire entre le piston (4) et le cylindre (2) est rempli par une phase liquide.

18. Procédé selon la revendication 17, **caractérisé en ce que,** après la désorption des radionucléides dans la phase liquide, le piston (4) est retiré hors du cylindre (2).

(8)

(7)

(13)

(9)

(10)

(1)

(2)

(3)

(12)

(17)

(6)

(15)

(16)

(5)

(11)

(4)

(8)

Fig. 1

Fig. 2

(5)　　　　　　　　　　　　(2)

(4)

(14)

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19500428
- US 5770030 A
- JP 2001281131 A